# EUROPEAN PATENT SPECIFICATION

(11) **EP 0 460 253 B1**
(45) Date of publication and mention of the grant of the patent: **03.08.1994**
(21) Application number: 90110653.4
(22) Date of filing: 06.06.1990
(51) Int. Cl.: C07C 67/26, C07C 69/54

(54) **Process for making carboxylic acid esters using a crosslinked acrylic resin as the catalyst**
Verfahren zur Herstellung Carbonsäureestern unter Anwendung eines vernetzten Acrylharzes als Katalysator
Procédé de fabrication d'esters d'acides carboxyliques utilisant une résine acrylique réticulée comme catalyseur

(43) Date of publication of application: 11.12.1991
(73) Proprietor: THE DOW CHEMICAL COMPANY, Midland, Michigan 48640 (US)
(72) Inventor: Rabon, James A., West Columbia, Texas 77486 (US); Pike, William C., Midland, Mich 48640 (US); Clinton, J. Boriack, Freeport, Texas 77541 (US)
(74) Representative: Sternagel, Hans-Günther, Dr.

(56) References cited:
- EP-A- 0 189 247
- US-A- 3 804 884

## Description

The present invention relates to a process for reacting a carboxylic acid with an epoxide to form a hydroxyalkyl ester by conducting the reaction in the presence of an anion exchange resin comprising the step of employing as the exchange resin a strong base macroporous anion exchange resin having an acrylic backbone.

More particularly, the present invention relates to the use of a macroporous strong base anion exchange acrylic-based resin as a catalyst for the production of hydroxyalkyl esters, via the reaction of an epoxide with a carboxylic acid. The resin catalyst of the present invention has been shown to give improved activity when compared to a styrene-divinylbenzene-based strong base anion exchange resin having an equivalent dry weight exchange capacity.

The preparation of esters by reacting an epoxide with a carboxylic acid employing as a catalyst, a strongly basic anion exchange resin, is well known technology. The preparation of hydroxyalkyl(meth)acrylates by reacting an alkylene oxide and acrylic or methacrylic acid is also known. The strongly basic anion exchange resin commonly employed as a catalyst in this reaction is a macroporous resin of the styrene-divinylbenzene type. The resin may be added to the reactor in any of several forms, e.g., as either the chloride or hydroxide form. U.S. Patent No. 3,340,295 describes the use of such a resin. A resin having a particular swelling capacity in water, a particular ion exchange capacity, and a limited amount of mono-substituted aromatic rings in the polymer backbone for catalyzing the subject reaction is described in U.S. Patent No. 3,804,884.

Strongly basic anion exchange resins have also been made from polymers having an acrylic backbone, the manufacture of which is found in numerous patents. One of the earliest of these patents is U.S. Patent No. 2,630,427, which teaches reacting in aqueous medium a tertiary amine, e.g., trimethylamine, with an insoluble, cross-linked copolymer of a glycidyl ester with a copolymerizable monomer containing two or three non-conjugated vinylidene groups.

U.S. Patent No. 2,862,893 discloses reacting the amine with a copolymer of a chlorohydrin ester and a copolymerizable monomer which contains at least two non-conjugated vinylidene groups.

U.S. Patent 3,340,295 teaches that the esterification reaction by means of reacting an alkylene oxide with a carboxylic acid is accomplished by employing numerous anion exchange resins, all of which are aromatic or cyclic in character. Ion exchange resins that are mentioned as suitable catalysts are products of the reaction of formaldehyde and heterocyclic tertiary nitrogen bases, e.g., N-substituted guanadines, with or without added phenol, and by the copolymerization of chloromethylated styrene and divinylbenzenes followed by amination with tertiary amines.

U.S. Patent No. 3,427,262 teaches crosslinking the polyacrylic structure with an aliphatic or cycloaliphatic hydrocarbon containing two or more allyl groups, e.g., hexa-1,5-diene, and, optionally, an additional crosslinking agent of a di- or trivinyl aromatic compound. The product is said to be more stable and less hydrolyzable.

U.S. Patent No. 3,551,357 discloses a process for making a hydrolysis-resistant acrylic ester-based anion exchange resin using other divinyl aromatic crosslinkers. Additionally, U.S. Patent No. 4,052,343 discloses a polymer which has ester functionality, and U.S. Patent No. 4,082,701 discloses a polymer having amide rather than ester functionality which is more resistant to hydrolysis.

These strongly basic acrylic-based anion exchange resins have utility in applications such as, for example, decolorizing cane sugar syrups (U.S. Patent No. 3,791,866), recovery of uranium complexes (U.S. Patent No. 3,870,663), and removal of dyes from waste streams and chemical and oxygen demanding wastes from pulp mill effluents (U.S. Patent No. 4,082,701).

The strong base anion exchange resins having an acrylic backbone and useful in the esterification reaction of the present invention include, for example, the macroporous anion exchange resins designated Amberlite™ (a trademark of Rohm & Haas Company) IRA-958 and Lewatit™ (a trademark of Mobay Corporation) AP-247-A which are commercially available. Such macroporous resins can have a dry weight exchange capacity (DWC) of from 1.5 to 4.4 milliequivalents (meq) of exchangeable chloride per gram of dried resin. Such macroporous resins are crosslinked with from 2 to 10 percent of a crosslinking monomer. The crosslinking monomer used herein can be, for example, ethylene glycol dimethacrylate; 1,4-butanediol diacylate; 1,4-butanediol dimethacrylate; triethylene glycol dimethacrylate; tetraethylene glycol dimethacrylate; diethylene glycol diacrylate; dielhylene glycol dimethacrylate; neopentyl glycol dimethacrylate; 1,6-hexanediol dimethacrylate; tetraethylene glycol diacrylate; triethylene glycol diacrylate; 1,3-butylene glycol dimethacrylate; 1,3-butylene glycol diacrylate; 1,10-decamethylene glycol diacrylate; 1,10-decamethylene glycol dimethacrylate; and mixtures thereof. When the macroporous resins are employed as catalysts in the esterification reaction of the present invention, the activity of such resins under certain specified conditions will provide at least about a 60 percent conversion of acid with greater than 98 percent selectivity to hydroxyalkyl acrylate.

Outside the dry weight exchange capacities of from 1.5 to 4.4 meq indicated above, the rate of reaction decreases, either because the number of reactive sites is too small at below 1.5 meq of the DWC range or because the sites are too crowded and unavailable to the reactants at above 4.4 meq of the DWC range. Outside the crosslinked range of from 2 to 10 percent indicated above, the resins either lose swelling ability at above 10 percent crosslinking or lose mechanical properties at below 2 percent crosslinking.

Epoxides which can be employed as reactants in the esterification reaction include, for example, alkylene oxides such as ethylene oxide (EO), propylene oxide (PO), 1,2- or 2,3-butylene oxides, hexene, cyclohexene and octene oxides and epoxides such as epichlorohydrin and styrene oxide.

Acids which can be esterified using the anion exchange resin catalysts of the invention are the unsaturated mono- and dicarboxylic acids having from 3 to 5 carbon atoms, for example, acrylic acid (AA), methacrylic acid, fumaric acid, maleic acid and itaconic acid. Saturated monocarboxylic acids having from 2 to 20 carbon atoms, for example, acetic acid, propanoic acid, butanoic acid, hexanoic acid, capric acid (10 carbons), myristic acid (14 carbons), palmitic acid (16 carbons) and stearic acid (18 carbons) and dicarboxylic acids having from 2 to 12 carbon atoms, for example, oxalic acid, succinic acid and adipic acid are useful in the present invention. Aromatic mono- and dicarboxylic acids such as benzoic acid and isomeric phthalic acids can also be esterified by the process of the present invention.

Molar ratios of epoxide to acid in the feed to a reactor used in the present invention are operable in a range of from 1.2:1 to 20:1, but ratios of from 3:1 to 12:1 are preferred. The molar ratio in the reactor, however, will change as the acid reactant is used up, the ratio becoming ever higher in favor of the oxide reactant. In a continuous reaction, the mole ratio of the reactants in the reactor will be determined by feed ratios and the particular conversion achieved.

The esterification reaction can be conducted in a stirred-batch reactor or in a fixed or fluid bed reactor. The reaction is conducted at a temperature of from 50°C to 110°C, preferably from 60°C to 90°C. Below the temperature of 50°C, the reaction becomes impractically slow and above the temperature of 110°C, polymerization of the reactants and/or product becomes a competing reaction.

Autogenous pressure, which depends upon the particular reactants and temperature at which the reaction is conducted, is generally employed in the esterification reaction of the present invention. The pressure employed, however, must be sufficient to keep the reactants in a liquid phase. Thus, esterification reactions using lower molecular weight epoxide such as ethylene oxide (Fw 44), propylene oxide (Fw 58) and isomeric butylene oxides (Fw 72) having boiling points which range from 13.5 to 63°C are most conviently run employing pressurized reactors. When higher molecular weight epoxides such as epichlorohydrin (Fw 92.5, b.p. 116°) or 1,2-epoxyhexane (Fw 100, b.p. 119°) are employed as reactants the exterifications are conveniently run at ambient pressure.

The reaction may be conducted in a solvent, although the excess of epoxide generally serves as a diluent. Solvents suitable for the reaction include inert aliphatic or aromatic hydrocarbons, for example, hexane, petroleum ether and xylene.

The strong base anion exchange resin employed as the catalyst in the present invention may be fed to the reactor in the form of a halide, hydroxide, alkoxide or carboxylic acid salt, but the resin is preferably used in the form of the chloride or of the carboxylate anion of the particular acid employed as reactant.

The following are representative examples of the invention, together with comparative examples using styrene-divinylbenzene anion exchange resins of the art. These examples were conducted in a batch reactor in which hydroxypropylacrylate (HPA) or hydroxyethylacrylate (HEA) was added to simulate the composition of a reaction mixture one would encounter in a continuous process for producing those esters. In all examples, both of the invention and comparative, the resin was dried at 50°C under vacuum for 18 hours prior to use in the reaction.

### Example 1

Into a stainless steel cylinder (300 ml), fitted with a rupture disc and appropriate valves, was introduced 5.0 g of died strong base anion exchange resin (Amberlite™ IRA-958) as a catalyst, 5.0 g of acrylic acid, hydroxypropyl acrylate (HPA) and 142 g of propylene oxide. The catalyst had a 4.1 dry weight exchange capacity (DWC). The cylinder was sealed and placed in a shaker bath at a temperature of 80°C and the contents in the cylinder were allowed to react for 40 minutes. The cylinder was then removed from the shaker bath and opened. The reaction mixture was drained from the cylinder without removing the catalyst resin beads from the cylinder. This procedure of charging the reactor with AA, HPA and PO, reacting for 40 minutes, and drawing the reaction product from the reactor was then repeated four times to allow the catalyst to age and the activity of the catalyst was measured by AA conversion to reach a constant level. After each run the reaction mixture was stripped of excess propylene oxide (PO) under vacuum and any amount of residual acid determined by titration with a standard sodium hydroxide solution. A reaction made after the catalyst had reached constant activity using 5.0 g acrylic acid (AA), 42.6 g HPA and 142.2 g PO, indicated that 65.7 percent of the AA had been converted to HPA.

### Comparative Example A

In a comparative experiment, made in the same manner as in Example II except for the use of a strong base styrene-divinylbenzene anion exchange resin which had the same DWC, i.e., 4.1, the conversion of AA to HPA was only 41.8 percent.

### Example 2

In this example an anion exchange resin having an acrylic backbone (Lewatit™ AP-247-A) was employed as the catalyst. The procedure was the same as in Example 1, again employing 5.0 g of dry catalyst, AA, PO and HPA. The catalyst was 8 percent crosslinked and had a DWC of 2.1. The reaction mixture, in the presence of the catalyst, was heated at a temperature of 80°C for 40 minutes. The liquid product was drained from the reactor retaining the catalyst in the reactor. The process of charging the reactor with AA, PO, and HPA, reacting for 40 minutes, and draining the reaction product from the reactor was repeated three additional times. The liquid product of these four runs was stripped of excess PO, and the product titrated for unreacted AA. The amount of AA conversion being used as a measure of catalyst activity. After the forth cycle of charging fresh reactants to the reactor, the catalyst had reached constant activity and the reactor was charged for a fifth time employing 5.0 g (0.069 M) AA, 42.75 g (0.329 M) HPA and 142.05 g (2.45 M) PO. The conversion of AA to HPA was 53.9 percent.

### Example 3

An esterification was conducted to make hydroxyethyl acrylate (HEA). Using the same strong base anion exchange resin (Amberlite IRA-958) and the same general procedure as in Example 1, accept HEA (48.18 g, 0.415 mol), AA (6.3 g, 0.087 mol) and ethylene oxide (EO) (143.3 g, 3.26 mol) were reacted in the presence of 12.8 g of the dried resin for 20 minutes at 68°C. The liquid product was drained from the reactor retaining the catalyst in the reactor. The process of charging the reactor with HEA, AA, and EO, reacting for 20 minutes and draining the reactor was repeated three additional times. The liquid product of these four resins was stripped of excess EO and the product analyzed for residual AA. The amount of AA conversion being used as a measure of catalyst activity. After the forth run above, the catalyst had reached constant activity and the reactor was charged for a fifth time employing the amounts of reactants and catalyst indicated above. The conversion of AA to HEA was 59.3 percent.

## Claims

1. A process of reacting a carboxylic acid with an epoxide to form a hydroxyalkyl ester by conducting the reaction in the presence of an anion exchange resin comprising the step of employing as the exchange resin a strong base macroporous anion exchange resin having an acrylic backbone.

2. The process of Claim 1 wherein the dry weight exchange capacity of said anion exchange resin is from 1.5 to 4.4 meq/g.

3. The process of Claim 1 wherein the anion exchange resin is crosslinked with from 2 to 10 percent of a crosslinking monomer.

4. The process of Claim 1, 2 or 3 wherein the epoxide is employed in a molar excess.

5. The process of Claim 4 wherein the mole ratio of the epoxide to the carboxylic acid is in the range of 1.2:1 to 20:1.

6. The process of any one of the preceding claims wherein the epoxide is an alkylene oxide having from 2 to 8 carbon atoms.

7. The process of any one of the preceding claims wherein the carboxylic acid is an unsaturated acid having from 3 to 6 carbon atoms.

8. The process of Claim 6 wherein the alkylene oxide is selected from ethylene oxide, propylene oxide, or butylene oxide, and the unsaturated acid is selected from acrylic acid or methacrylic acid.

9. The process of Claim 6, 7 or 8 wherein the molar ratio of alkylene oxide to unsaturated acid is from 3:1 to 12:1.

## Patentansprüche

1. Verfahren zum Umsetzen einer Carbonsäure mit einem Epoxid, um einen Hydroxyalkylester auszubilden durch Ausführen der Umsetzung in Gegenwart eines Anionenaustauscherharzes, wobei als Austauscherharz ein stark basisches makroporöses Anionenaustauscherharz mit einer Acrylhauptkette verwendet wird.

2. Verfahren nach Anspruch 1,
**dadurch gekennzeichnet,**
daß die trockene Gewichtsaustauschkapazität des Anionenaustauscherharzes von 1,5-4,4 meq/g beträgt.

3. Verfahren nach Anspruch 1,
**dadurch gekennzeichnet,**
daß das Anionenaustauscherharz mit 2-10 % eines vernetzenden Monomeren vernetzt ist.

4. Verfahren nach Anspruch 1, 2 oder 3,
**dadurch gekennzeichnet,**
daß Epoxid in molarem Überschuß verwendet wird.

5. Verfahren nach Anspruch 4,
**dadurch gekennzeichnet,**
daß das Molverhältnis von Epoxid:Carbonsäure im Bereich von 1,2:1 bis 20:1 liegt.

6. Verfahren nach einem der vorstehenden Ansprüche,
**dadurch gekennzeichnet,**
daß das Epoxid ein Alkylenoxid mit 2-8 Kohlenstoffatomen ist.

7. Verfahren nach einem der vorstehenden Ansprüche,
**dadurch gekennzeichnet,**
daß die Carbonsäure eine ungesättigte Säure mit 3-6 Kohlenstoffatomen ist.

8. Verfahren nach Anspruch 6,
**dadurch gekennzeichnet,**
daß das Alkylenoxid ausgewählt ist aus Ethylenoxid, Propylenoxid oder Butylenoxid und die ungesättigte Säure aus Acrylsäure oder Methacrylsäure ausgewählt ist.

9. Verfahren nach Anspruch 6, 7 oder 8,
**dadurch gekennzeichnet,**
daß das Molverhältnis von Alkylenoxid zu ungesättigter Säure von 3:1 bis 12:1 beträgt.

## Revendications

1. Procédé de réaction d'un acide carboxylique avec un époxyde afin de former un ester d'hydroxyalkyle en effectuant la réaction en présence d'une résine échangeuse d'anions comprenant l'étape consistant à employer comme résine échangeuse une résine échangeuse d'anions macroporeuse fortement basique à chaîne principale acrylique.

2. Procédé selon la revendication 1 dans lequel la capacité d'échange rapportée au poids sec de ladite résine échangeuse d'anions est comprise entre 1,5 et 4,4 meq/g.

3. Procédé selon la revendication 1 dans lequel la résine échangeuse d'anions est réticulée avec 2 à 10 pour-cent d'un monomère réticulant.

4. Procédé selon la revendication 1, 2 ou 3 dans lequel l'époxyde est employé avec un excès molaire.

5. Procédé selon la revendication 4 dans lequel le rapport molaire entre l'époxyde et l'acide carboxylique est compris entre 1,2:1 et 20:1.

6. Procédé selon l'une quelconque des revendications précédentes dans lequel l'époxyde est un oxyde d'alkylène à 2 à 8 atomes de carbone.

7. Procédé selon l'une quelconque des revendications précédentes dans lequel l'acide carboxylique est un acide non saturé à 3 à 6 atomes de carbone.

8. Procédé selon la revendication 6 dans lequel l'oxyde d'alkylène est choisi parmi l'oxyde d'éthylène, l'oxyde de propylène, ou l'oxyde de butylène, et l'acide non saturé est choisi parmi l'acide acrylique et l'acide méthacrylique.

9. Procédé selon la revendication 6, 7 ou 8 dans lequel le rapport molaire entre l'oxyde d'alkylène et l'acide non saturé est compris entre 3:1 et 12:1.
